# EUROPEAN PATENT SPECIFICATION

(11) **EP 1 937 287 B1**
(45) Date of publication and mention of the grant of the patent: **29.02.2012**
(21) Application number: 06777200.4
(22) Date of filing: 28.09.2006
(51) Int. Cl.: A61K 33/26, A61K 33/34, A23L 1/304

(54) **MIXTURE OF IRON AND COPPER SALTS MASKING METALLIC TASTE**
GEMISCH AUS EISEN UND KUPFERSALZEN ZUR MASKIERUNG DES METALLISCHEN GESCHMACKS
MÉLANGE DE SELS DE FER ET DE CUIVRE MASQUANT UN GOÛT MÉTALLIQUE

(30) Priority: 11.10.2005 EP 05292120
(43) Date of publication of application: 02.07.2008
(73) Proprietor: Bayer Consumer Care AG, 4052 Basel (CH)
(72) Inventor: GARCIN, Patrice, F-74890 Bons En Chablais (FR); KABARADJIAN, Catherine, F-74100 Vétraz-Monthoux (FR)
(74) Representative: Albers, Markus
(86) International application number: PCT/EP2006/009434
(87) International publication number: WO 2007/042153

(56) References cited:
- GB-A- 1 322 102
- US-A- 5 211 940
- US-A- 5 459 162
- US-A1- 2005 037 065
- US-A1- 2005 170 014
- US-B1- 6 368 621
- US-B1- 6 521 247
- REDDY ET AL: "Evaluation of efficacy and safety of iron polymaltose complex and folic acid (Mumfer) vs iron formulation (ferrous fumarate) in female patients with anaemia." JOURNAL OF THE INDIAN MEDICAL ASSOCIATION, vol. 99, no. 3, 2001, pages 154-155, XP009060053
- PATENT ABSTRACTS OF JAPAN vol. 013, no. 431 (C-640), 26 September 1989 (1989-09-26) & JP 01 168611 A (LION CORP), 4 July 1989 (1989-07-04)

## Description

The present invention relates to a novel oral nutritional composition without metallic taste comprising an iron source and a copper source, its process for preparation and its use for balancing the provision of iron as nutritional supplement.

Iron is essential to most life forms and to normal human physiology. Iron is an integral part of many proteins and enzymes that maintain good health. In humans, iron is an essential component of proteins involved in oxygen transport (Dallman PR. Biochemical basis for the manifestations of iron deficiency. Annu Rev Nutr 1986, 6, 13-40). It is also essential for the regulation of cell growth and differentiation (Andrews NC. Disorders of iron metabolism. N Engl J Med 1999, 341, 1986-95). A deficiency of iron limits oxygen delivery to cells, resulting in fatigue, poor work performance, and decreased immunity (Haas JD, Brownlie T 4th. Iron deficiency and reduced work capacity: a critical review of the research to determine a causal relationship. J Nutr 2001, 131, 691S-6S, Bhaskaram P. Immunobiology of mild micronutrient deficiencies. Br J Nutr 2001, 85, S75-80).

Several oral compositions are known for the use as nutritional or dietary supplement containing iron.

Reddy et al. compare an iron polymaltose complex and ferrous fumarate for the "Evaluation of efficacy and safety of iron polymaltose complex and folic acid (Mumfer) vs. iron formulation (ferrous fumarate) in female patients with anaemia" in the Journal of the Indian Medical Association, Vol. 99, no.3, 2001, p. 154-155.

GB 1322102 A describe oral compositions comprising iron polymaltose for growth stimulation and treatment of anemia in piglets.

US 6,3683621 B1 discloses oral compositions comprising iron polymaltose and unsaturated fatty acids for pregnant and breast-feeding women.

US 2005/170014 A1 describes a slowly dissolving controlled release formulation comprising carbonyl iron for the treatment of anemia.

US 2005/037065 A1 discloses soft gel capsules comprising essential fatty acids and carbonyl iron for pregnant and breast-feeding women.

US 6,521,247 B1 describes coated formulations comprising carbonyl iron as slowly dissolving iron compound combined with a fast dissolving iron compound and further minerals and vitamins for administration as prenatal supplement, during pregnancy and lactation.

US 5,459,162 A discloses a nutritional copper citrate comprising supplement for chicken, but no iron compound.

JP 01168611 A and US 5,211,940 A refer to copper citrate comprising mouth wash compositions. No iron compound is mentioned.

Other known compositions are e.g. multi-vitamin and mineral supplements or oral dosage forms containing a higher dose of iron for treating iron deficiency symptoms. However, in order to avoid a bad metallic taste the amount of iron is limited and the use of a complex and expensive taste masking technology such as coating of iron containing granules or tablets, admixing taste masking flavours, use of capsules is necessary.

The object of the present invention is to provide an alternative oral composition containing an iron source and a copper source having no metallic bad taste and avoiding a complex and expensive taste masking technology.

Surprisingly it is found that a metallic bad taste of the composition according to the present invention can be avoided.

Subject of the present invention is an oral nutritional supplement without taste masking technology comprising an iron source and a copper source, whereby the iron source is carbonyl iron, and the copper source is copper citrate or whereby the iron source is ferric pyrophosphate, and the copper source is copper citrate in an effervescent tablet according to example 8.

The oral composition according to the invention has not a metallic bad taste, in particular aftertaste.

The iron source iron polymaltose is a water soluble macromolecular complex of polynuclear iron (III) hydroxide and partially hyrdolysed dextrin (polymaltose). Iron polymaltose is known and described in Martindale 32nd edition, 12864-h, p 1349.

The iron source carbonyl iron is a form of special purified elemental iron having a mean particle size of less than 7 pm. Carbonyl iron is known under the CAS number 7439-89-6, BP73.

According to the present invention copper citrate includes any kind of solvate such as hydrates. Preferably the hemipentahydrate of copper citrate is used.

The amount of the iron source in the composition according to the invention is from 1 to 60 mg, preferably, from 2 to 20 mg based on iron.

The amount of the copper citrate in the composition according to the invention is from 0.1 to 2 mg, preferably 0.3 to 1.1 mg based on copper.

In particular the ratio between iron and copper in the composition is from 1:2 to 50:1, preferably from 5:1 to 25:1, most from 10:1 to 20:1. "Ratio" means for the purposes of the invention the ratio by weight of the individual components based on iron and copper.

The composition according to the invention can comprise further active ingredients such as vitamins and minerals. Vitamins include, but are not limited to, vitamin A, beta carotene, vitamin C (ascorbic acid), vitamin D3 (cholecalcipherol), vitamin E (tocopherol acetate), vitamin B1 (thiamine), vitamin B2 (riboflavin), nicotinamide, vitamin B5 (panthothenic acid), vitain B6 (pyridoxine), folic acid, vitamin B12 (cyanocobalamin), vitamin K1 and biotin. Minerals include, but are not limited to, calcium salts such as calcium carbonate, calcium phosphate, calcium glycerophosphate; magnesium salts such as magnesium phosphate or magnesium oxide; zinc salts such as zinc citrate; selenium salts such as sodium selenate; potassium iodide; manganese salts such as manganese sulphate; molybdate salts such as sodium molybdate; chrom salts such as chromium chloride; sodium chloride and potassium chloride.

Furthermore the composition according to the invention can also comprise in additon iron fumarate 60% Descote. A preferred composition comprises copper citrate and iron fumarate 60% Descote.

Iron fumarate 60% Descote contents 60% iron fumarate coated with 40% mono- and di-glycerides (Merck index 12th edition, p4087, ref 4094, Martindale 32nd edition, p 1339 ref 5054).

Subject of the present invention is an oral nutritional composition without taste masking technology comprising an iron source which is carbonyl iron, and copper citrate and optionally at least one further mineral and/or vitamin.

The composition according to the invention is administered orally one or more, preferably up to three, more preferably up to two times per day. With each administration the number of dosage forms taken in at the same time should not exceed two.

The composition according to the present invention can be used as nutritional supplement or as dietary supplement for balancing the supply of iron in a patient. A patient, for the purpose of this invention, is a mammal, including a human.

Nevertheless, it may in some cases be advantageous to deviate from the amounts specified, depending on body weight, individual behaviour toward the active ingredient, type of preparation and time or interval over which the administration is effected. For instance, less than the aforementioned minimum amounts may be sufficient in some cases, while the upper limit specified has to be exceeded in other cases.

The composition according to the invention comprises suitable administration forms which deliver the compound of the invention and which include, but are not limited to, tablets, tablets which disintegrate rapidly in the oral cavity (orodispersible tablets), powders, sachets, granules, pellets, chewable tablets, chewing gums, dispersible tablets, effervescent compositions such as for example effervescent tablets or effervescent granules, liquids, solutions, emulsions, gels, syrups or drops.

Preference is given to tablets, granules, orodispersible tablets, chewable tablets, chewing gums, dispersible tablets, effervescent tablets and effervescent granules. More preferably the application forms are chewable tablets, orodispersible tablets, chewing gums, dispersible tablets, effervescent tablets and effervescent granules.

Ingredients of the oral dosage forms are those which are accepted for pharmaceuticals and nutritional supplements and physiologically unobjectionable, for example: as fillers cellulose derivatives (e.g. microcrystalline cellulose), sugars (e.g. lactose), sugar alcohols (e.g. mannitol, sorbitol), inorganic fillers (e.g. calcium phosphates), binders (e.g. polyvinylpyrrolidone, gelatin, starch derivatives and cellulose derivatives), and all other excipients required to produce formulations of pharmaceuticals and nutritional supplements of the desired properties, e.g. lubricants (magnesium stearate), e.g. disintegrants (e.g. crosslinked polyvinylpyrrolidone, sodium carboxymethylcellulose), e.g. wetting agents (e.g. sodium lauryl sulphate), e.g. release-slowing agents (e.g. cellulose derivatives, polyacrylic acid derivatives), e.g. stabilizers, e.g. sweeteners, e.g. antioxidants, e.g. preservatives e.g. flavourings, e.g. coloured pigments, e.g. effervescent couples preferably citric acid as acid component and sodium carbonate and/or sodium hydrogen carbonate as basic component).

Liquid formulations are likewise produced by a standard method using excipients which are usual for pharmaceuticals and nutritional supplements and contain the active ingredients either dissolved or suspended. Typical administration volumes of these pharmaceutical and nutritional supplement preparations are 1 to 10 ml. Examples of excipients in these liquid formulations are: solvents (e.g. water, alcohol, natural and synthetic oils, e.g. medium chain-link triglycerides), solubilizers (e.g. glycerol, glycol derivatives), wetting agents (e.g. polysorbate, sodium lauryl sulphate), and further excipients required to produce formulations of pharmaceuticals and nutritional supplements of the desired properties, e.g. viscosity-increasing agents, e.g. pH-correcting agents, e.g. sweeteners and flavourings, e.g. antioxidants, e.g. stabilizers, e.g. preservatives.

Excipients for pharmaceuticals and nutritional supplements familiar to the skilled person are also described for example in the following handbook: "Handbook of Pharmaceutical Excipients", Wade, A. & Weller, P.J., American Pharmaceutical Association, Washington, 2nd edition 1994.

The dosage forms mentioned herein are produced by general standard processes. E.g. Tablets or chewable tablets can be produced by mixing and/or granulating the active ingredients together with the excipients to form a blend which is finally pressed to tablets. Optionally different blends containing different ingredients and excipients can be premixed and combined to a final blend which is then pressed to tablets. In the case of an effervescent formulation the acid/base couple can be add e.g. to final blend or the acid and the base are added at different times to the blend. Also the base and the acid can be added to different blends which are finally combined.

Advantage of the composition of the present invention is that for the preparation of the composition a complex and expensive taste masking technology known in the prior art such as coating of tablets or coating of granules or putting the iron component into a capsule is not needed. The composition of the present invention can be prepared by simple and well-known standard procedures. Another well-known taste masking method is the addition of flavours in order to cover and mask the bad taste. This taste masking method is normally restricted to only a few applicable flavours which have to be selected in each case. However, flavouring ingredients are not needed for taste masking in the composition of the present invention.

Subject of the present invention is an oral nutritional composition without taste masking technology comprising an iron source which is carbonyl iron, and copper citrate as copper source which has no metallic taste in particular after taste and which is not a coated tablet, coated granule or capsule.

Subject of the present invention is an oral nutritional composition without taste masking technology comprising an iron source which is carbonyl iron, and copper citrate as copper source and optionally at least one further mineral and/or vitamin wherein the composition has no metallic taste and is not a coated tablet, coated granule or capsule.

Preference is given to a fast disintegrating orodispersible tablet. The disintegration time is less equal than 100 sec., preferably less equal than 80 sec.

### Examples:

### Example 1 (according to the invention):

| Chewable tablet | | Tablet weight 1.230 g | |
|---|---|---|---|
| **Active ingredients** | **Amount based on the active ingredient [mg]** | **Form of the active ingredient** | **Amount of the form of the active ingredient [mg]** |
| vit A | 400 | vitamin A palmitate 100000 iu/g | 13,33 |
| vit C | 30 | ascorbic acid | 30 |
| Vit D3 | 2,5 | cholecalciferol 100000 IU/g | 0,8 |
| vit E | 5 | tocopherol acetate 50% CWS/S | 14 |
| thiamin (B1) | 1,05 | thiamin nitrate 33% Rocoat | 3,9 |
| riboflavin (B2) | 1,2 | riboflavine 33% Rocoat | 3,6 |
| nicotinamide (PP) | 13,5 | nicotinamide | 13,5 |
| pantothenic acid (B5) | 3 | calcium pantothenate | 3,26 |
| pyridoxine (B6) | 1,5 | pyridoxine hydrochloride 33% Rocoat | 5,46 |
| folic acid | 150 | folic acid 10% trituration | 1,5 |
| Cyanocobalamin (B12) | 0,75 | dry vit B120,1% WS | 0,75 |
| biotin | 75 | biotin 1% trituration | 7,5 |
| | | | |
| calcium | 60 | calcium carbonate DCCS90L | 166,53 |
| magnesium | 25 | magnesium oxide | 41,45 |
| phosphorus | | | |
| iron | 4 | Carbonyl iron | 4,08 |
| zinc | 3,75 | Zinc citrate trihydrate | 12,02 |
| iodine | 37,5 | potassium iodide 5% trituration | 0,75 |
| selenium | 25 | sodium selenate anhydrous 1% trituration | 2,5 |
| copper | 0,45 | copper citrate 2,5 H₂O | 1,28 |
| manganese | 0,9 | manganese sulfate monohydrate | 2,77 |
| molybdenum | 22,5 | sodium molybdate dihydrate 1% trituration | 2,25 |
| chromium | 12,5 | chromic chloride hexahydrate 1% trituration | 1,25 |

Further excipients can be added to example 1:

| **excipients** | **Amount [mg]** |
|---|---|
| xylitol | 75 |
| talc | 50 |
| anhydrous citric acid | 20 |
| magnesium stearate | 10 |
| orange flavour | 10 |
| Passion fruit flavour | 5 |
| aspartam | 5 |
| sucralose | 1,5 |
| iron oxide | 3 |

### Example 2 (according to the invention):

| Effervescent tablet | | | |
|---|---|---|---|
| **Active ingredients** | **Amount based on the active ingredient [mg]** | **Form of the active ingredient** | **Amount of the form of the active ingredient [mg]** |
| vit A | 595 | vitamin A palmitate 100000 iu/g | 13,33 |
| beta caroten | 0,63 | betatab 10%E | 0,63 |
| vit C | 180 | ascorbic acid | 30 |
| Vit D3 | 5 | cholecalciferol 100000 IU/g | 0,8 |
| vit E | 10 | tocopherol acetate 50% CWS/F | 14 |
| vit K | 0,03 | vitamin K 15% SD | |
| thiamin (B1) | 4,2 | thiamin phosphate | 3,9 |
| riboflavin (B2) | 4,8 | riboflavine phosphate | 3,6 |
| nicotinamide (PP) | 54 | nicotinamide | 13,5 |
| pantothenic acid (B5) | 18 | calcium pantothenate | 3,26 |
| pyridoxine (B6) | 6 | pyridoxine hydrochloride 33% Rocoat | 5,46 |
| folic acid | 600 | folic acid 10% trituration | 1,5 |
| Cyanocobalamin (B12) | 0,003 | dry vit B12 0,1% WS | 0,75 |
| biotin | 0,03 | biotin 1% trituration | 7,5 |
| calcium | 120 | calcium carbonate | 71,7 |
| | | calcium phosphate dibasic anhydrous | 151,38 |
| | | calcium glycerophosphate | 259,86 |
| magnesium | 45 | magnesium phosphate dibasic trihydrate | 322,81 |
| phosphorus | 126,3 | see Ca & Mg | |
| iron | 18 | Carbonyl iron | 18,33 |
| zinc | 8 | Zinc citrate trihydrate | 25,63 |
| iodine | 0,075 | potassium iodide | 0,098 |
| selenium | 0,055 | sodium selenate | 0,132 |
| copper | 0,9 | copper citrate | 2,57 |
| manganese | 1,8 | manganese sulfate monohydrate | 5,54 |
| molybdenum | 0,045 | sodium molybdate dihydrate | 0,113 |
| chromium | 0,025 | chromium chloride hexahydrate | 0,128 |
| chloride | 39,8 | sodium chloride | 30 |
| potassium | 20,4 | potassium chloride | 43,51 |

Further excipients can be added to example 2:

| **excipients** | **Amount [mg]** |
|---|---|
| maltitol | qs |
| sorbitol | qs |
| sodium hydrogen carbonate | 950 |
| anhydrous citric acid | 1800 |
| sodium carbonate | 65 |
| PVP | 30 |
| crospovidone | 7 |
| sucrose ester of fatty acids | 0,15 |
| beet red | 5 |
| orange flavour | 77 |
| Passion fruit flavour | 30 |
| aspartam | 35 |
| acesulfam K | 20 |

| | |
|---|---|
| qs: quantum satis =quantity to add to reach the tablet weight | |

### Example 3 (comparative example):

| Orodispersible tablet | | Tablet weight 1000 mg | |
|---|---|---|---|
| **Active ingredients** | **Amount based on the active ingredient [mg]** | **Form of the active ingredient** | **Amount of the form of the active ingredient [mg]** |
| vit A | 400 | vitamin A palmitate 100000 iu/g | 13,33 |
| vit C | 30 | ascorbic acid | 30 |
| Vit D3 | 2,5 | cholecalciferol 100000 IU/g | 0,8 |
| vit E | 5 | tocopherol acetate 50% CWS/F | 14 |
| thiamin (B 1) | 1,05 | thiamin nitrate 33% Rocoat | 3,9 |
| riboflavin (B2) | 1,2 | riboflavine 33% Rocoat | 3,6 |
| nicotinamide (PP) | 13,5 | nicotinamide | 13,5 |
| pantothenic acid (B5) | 3 | calcium pantothenate | 3,26 |
| pyridoxine (B6) | 1,5 | pyridoxine hydrochloride 33% Rocoat | 5,46 |
| folic acid | 150 | folic acid 10% trituration | 1,5 |
| Cyanocobalamin (B12) | 0,75 | dry vit B12 0,1% WS | 0,75 |
| biotin | 75 | biotin 1% trituration | 7,5 |
| | | | |
| calcium | 60 | calcium carbonate DCCS90L | 166,53 |
| magnesium | 25 | magnesium oxide | 41,45 |
| iron | 4 | iron fumarate 60% Descote | 20,28 |
| zinc | 3,75 | Zinc citrate trihydrate | 12,02 |
| iodine | 37,5 | potassium iodide 5% trituration | 0,75 |
| selenium | 25 | sodium selenate anhydrous 1% trituration | 2,5 |
| copper | 0,45 | copper citrate 2,5 H₂O | 1,28 |
| manganese | 0,9 | manganese sulfate monohydrate | 2,77 |
| molybdenum | 22,5 | sodium molybdate dihydrate 1% trituration | 2,25 |
| chromium | 12,5 | chromic chloride hexahydrate 1% trituration | 1,25 |
| pharmaburst C 1 | | | qs 1 tablet |
| anhydrous citric acid | | | 16 |
| magnesium stearate | | | 25 |
| sodium hydrogencarbonate | | | 20 |
| pineapple flavour | | | 3 |
| Passion fruit flavour | | | 3 |
| aspartam | | | 13 |
| iron oxide | | | 2,5 |

### Example 4: Manufacturing process for effervescent tablets

### (manufacturing process for examples 2)

### Vitamin Blend :

In a drum thiamine monophosphoric acid ester chloride dihydrate, rboflavine sodium phosphate, pyridoxine hydrochloride, sodium chloride and sorbitol (in part) are mixed together to the premix 1. Into the tank of the blender the following ingredients are introduced and mixed: premix 1, ascorbic acid, vitamin A palmitate, vitamin E powder, nicotinamide, betatab 10% E, calcium pantothenate, orange flavour, passion fruit flavour, anhydrous sodium carbonate, acesulfame potassium, aspartame, crospovidone, sorbitol (remaining part), beet red.

### Mineral Granule :

The following ingredients are mixed: copper citrate 2.5 H₂O, zinc citrate trihydrate, iron source, manganese sulphate monohydrate, calcium carbonate, maltitol, calcium hydrogen phosphate anhydrous (remaining part), calcium glycerophosphate, magnesium hydrogen phosphate trihydrate, sodium hydrogen carbonate and potassium chloride. Thereafter the mixure is granulated by spraying a binding solution made of purified water, sodium molybdate dihydrate, sodium selenate anhydrous, potassium iodide, ethanol, sucrose esters of fatty acids, povidone K90 and chromic chloride hexahydrate. The granule is dried, cooled and sieved.

### Final Blend :

In a drum folic acid, biotin, vitamin B12, vitamin K1, vitamin D3 and sorbitol (= premix 2) are mixed together. In the tank of the tumble mixer the mineral granule, the vitamin blend, the premix 2 and anhydrous citric acid are mixed to the final blend.

### Compression :

The homogeneous final blend is compressed to tablets on a rotary tablet press.

### Example 5: Manufacturing process for chewable tablets

### Premix:

In a drum dry vitamin D3, thiamine mononitrate, riboflavin, pyridoxine hydrochloride, folic acid 1, vitamin B12, calcium pantothenate, biotin, copper citrate 2.5 H₂O, manganese sulphate monohydrate, iodine, selenium, chromium, molybdenum, orange flavour, passion fruit flavour, iron oxide yellow, aspartame, sucralose NF micronized, pearlitol SD 200 are mixed for 15 minutes.

### Final blend:

Thereafter the premix, vitamin A palmitate, nicotinamide, dry vitamin E, ascorbic acid, calcium carbonate, magnesium oxide heavy, iron source, zinc citrate 3 H₂O, anhydrous citric acid, xylitol, talc, pearlitol SD 200 and sorbitol are mixed for 20 minutes. Magnesium stearate is added and the blend is mixed again for 5 additional minutes.

### Compression:

The homogeneous final blend is pressed to tablets on a rotary tablet press.

### Example 6: Manufacturing process for orodispersible tablets

### Premix:

In a drum dry vitamin D3, thiamine mononitrate, riboflavin, pyridoxine hydrochloride, folic acid, vitamin B12, calcium pantothenate, biotin, copper citrate 2,5 H₂O, manganese sulphate monohydrate, iodine, selenium, chromium, molybdenum, pineapple flavour, passion fruit flavour, iron oxide yellow, aspartame and pharmaburst C1 are mixed for 15 minutes.

### Final blend:

Premix, vitamin A palmitate, nicotinamide, dry vitamin E, ascorbic acid, calcium carbonate, magnesium oxide heavy, iron source, zinc citrate 3 H₂O, anhydrous citric acid, sodium hydrogen carbonate and pharmaburst C1 are mixed for 20 minutes. Thereafter magnesium stearate is added and it is mixed again for 5 additional minutes.

### Compression:

The homogeneous final blend is compress to tablets on a rotary tablet press.

### Example 7(comparative example): Tablet for children

| Better taste | Tablet weight 1230 mg | in form of | |
|---|---|---|---|
| claim | quantitative | qualitative | quantitive |
| vit A | 1000 | vitamin A palmitate 100000 iu/g | 10 |
| vit C | 22.5 | Acerola extract 25% vitamin C enriched | 90 |
| Vit D3 | 2,5 µg | cholecalciferol 100000 IU/g | 0,8 |
| vit E | 6 | tocopherol acetate 50% CWS/F | 17.9 |
| thiamin (B1) | 0.45 | thiamin nitrate 33% Rocoat | 1.60 |
| riboflavin (B2) | 0.45 | riboflavine 33% Rocoat | 1.27 |
| nicotinamide (PP) | 6 | nicotinamide | 6 |
| pantothenic acid (B5) | 2 | calcium pantothenate | 2.17 |
| pyridoxine (B6) | 0.45 | pyridoxine hydrochloride 33% Rocoat | 1.61 |
| folic acid | 75 µg | folic acid 10% trituration | 0.75 |
| Cyanocobalamin (B 12) | 1 µg | dry vit B12 0,1 % WS | 1 |
| biotin | 10 µg | biotin 1% trituration | 1 |
| Choline | 25 | Choline bitartrate | 60.75 |
| calcium | 120 | calcium carbonate DCCS90L | 335.30 |
| magnesium | 25 | magnesium oxide | 42,89 |
| iron | 6 | ferrous fumarate 60% Descote | 32.41 |
| zinc | 4 | Zinc citrate trihydrate | 12.62 |
| iodine | 60 µg | potassium iodide 5% trituration | 1.137 |
| selenium | 12.5 µg | sodium selenate anhydrous 1% trituration | 1.16 |
| copper | 0,40 | copper citrate 2,5 H₂O | 1,23 |
| manganese | 1 | manganese sulfate monohydrate | 3.09 |
| chromium | 12,5 µg | chromic chloride hexahydrate 1% trituration | 1,22 |
| Mannitol | | | ca. 200 |
| anhydrous citric acid | | | 20 |
| talc | | | 50 |
| magnesium stearate | | | 10 |
| xylitol | | | 75 |
| Sorbitol | | | ca. 200 |
| cherry flavour | | | 3 |
| Grenadine flavour | | | 3 |
| aspartam | | | 3 |
| sucralose | | | 0.8 |
| iron oxide | | | 2,5 |

### Example 8 (according to the invention):

| Effervescent tablet | | Tablet weight 4800 mg | |
|---|---|---|---|
| **Active ingredients** | **Amount based on the active ingredient** | **Form of the active ingredient** | **Amount of the form of the active ingredient** |
| vit A as retinol | (2667.7 IU) 800 µg | vitamin A palmitate 100000 IU/g | (2266.7 IU) 22.67 mg |
| | | beta carotene as betatab 10%E | (400 IU) 7.20 mg |
| vitamin D 3 | (200 IU) 5 µg | Cholecalciferol concentrate (water dispersible powder form) 100000 IU/g | 2.0 mg |
| Vitamin E | 10 mg | D-alpha-tocopherol acetate concentrate (powder form) 50 % | 29.80 mg |
| Vitamin K | 0.03 mg | vitamin K1 5% SD | 0.60 mg |
| Vitamin B1 | 4.20 mg | Thiamin monophosphoric acid ester chloride dihydrate | 6.59 mg |
| Vitamin B2 | 4.80 mg | Sodium riboflavin 5'-phosphate | 6.56 mg |
| Niacin | 54.0 mg | nicotinamide | 54.0 mg |
| Pantothenic acid | 18.0 mg | Calcium D-pantothenate | 19.57 mg |
| Vitamin B6 | 6.0 mg | Pyridoxine hydrochloride | 7.3 mg |
| Folic acid | 0.60 mg | Pteroylmonoglutamic acid | 0.60 mg |
| Vitamin B12 | 3.0 µg | Cyanocobalamin powder 0.1 % water soluble | 3.0 mg |
| Biotin | 30 µg | D-biotin | 30 µg |
| Vitamin C | 180 mg | Ascorbic acid fine powder | 180 mg |
| Calcium | 120 mg | Calcium carbonate | 295.61 mg |
| | | (see calcium pantothenate) | |
| Magnesium | 80 mg | Magnesium carbonate | 163 mg |
| | | Magnesium sulphate dihydrate | 159 mg |
| Iron | 14 mg | Ferric pyrophosphate | 56 mg |
| copper | 0.9 mg | Copper citrate 2.5 hydrate | 2.57 mg |
| iodine | 0.075 mg | Potassium iodine | 0.098 mg |
| zinc | 8 mg | Zinc citrate trihydrate | 25.63 mg |
| manganese | 1.8 mg | Manganese sulphate monohydrate | 5.54 mg |
| potassium | 20.4 mg | Potassium chloride | 38.96 mg |
| selenium | 50 µg | Sodium selenate anhydrous | 0.12 mg |
| chromium | 0.025 mg | Chromium chloride hexahydrate | 0.128 mg |
| molybdenum | 0.045 mg | Sodium molybdate dihydrate | 0.113 mg |
| Coenzyme Q10 | 3.0 mg | Coenzyme Q10 10 % | 30 mg |

Further excipients can be added to example 8:

| **excipients** | **Amount [mg]** |
|---|---|
| Mannitol 100 mesh ²⁾ | qs |
| Mannitol SD 200 ¹⁾ | qs |
| Sorbitol ¹⁾ | qs |
| Anhydrous citric acid | 1800 |
| Sodium hydrogen carbonate | 950 |
| Orange juice flavour | 77 |
| Sodium carbonate | 65 |
| Passion fruit flavour | 30 |
| aspartam | 35 |
| Potassium acesulfam | 20 |
| crospovidone | 7 |
| beet red | 5 |
| sucrose ester of fatty acids | 0.15 |

| | |
|---|---|
| qs: quantum satis =quantity to add to reach the tablet weight ¹⁾ Half of mannitol SD 200 and half of sorbitol are used to obtain the theoretical tablet weight of 4800 mg. ²⁾ The quantity of mannitol 100 mesh is adjusted according to mineral contents in the granule. | |

### Manufacturing process for example 8:

### Vitamin Blend :

In a drum vitamin D3 100 CWS, biotin, folic acid, vitamin B12 0.1% WS, vitamin K1 5% SD, coenzyme Q10 10% CWS/S, sodium molybdate dihydrate, sodium selenate anhydrous and mannitol (a part) are mixed together to the premix 1. Into the tank of the blender the following ingredients are introduced and mixed: premix 1, vitamin A palmitate, nicotinamide, calcium pantothenate, vitamin E powder, pyridoxine hydrochloride, riboflavin sodium phosphate, thiamin monophosphoric, betatab 10% E, crospovidone, beet red and mannitol (a part).

### Mineral Granule :

The following ingredients are mixed: copper citrate 2.5 H₂O, zinc citrate trihydrate, iron source, manganese sulfate monohydrate, calcium carbonate, magnesium carbonate, magnesium sulfate dihydrate, sodium hydrogen carbonate, potassium chloride, anhydrous citric acid and mannitol (rest). Thereafter the mixture is granulated by spraying a binding solution made of ethanol, sucrose esters of fatty acids, potassium iodide and chromium chloride hexahydrate. The granule is dried, cooled and sieved.

### Final Blend :

In the tank of the tumble mixer the mineral granule, the vitamin blend, ascorbic acid, sodium hydrogen carbonate, sorbitol, anhydrous sodium carbonate, sodium chloride, orange flavour, passion fruit flavour, potassium acesulfame, aspartame and mannitol SD 200 are mixed to the final blend.

### Tabletting :

The homogeneous final blend is compressed to tablets on a rotary tablet press.

### Results:

### Examples 1 to 3, 7 and 8 do not show a metallic bad taste when administered orally.

The disintegration time for example 3 prepared according to example 6 (orodispersible tablets) is 67 sec.. The disintegration time can be measured by standard methods known to the person skilled in the art.

## Claims

1. Oral nutritional supplement without taste masking technology comprising an iron source and a copper source, whereby the iron source is carbonyl iron, and the copper source is copper citrate.

2. Oral nutritional supplement without taste masking technology comprising an iron source and a copper source, whereby the iron source is ferric pyrophosphate, and the copper source is copper citrate in an effervescent tablet of about 4800 mg with the following composition of active ingredients:
| Active ingredients | Amount based on the active ingredient | Form of the active ingredient | Amount of the form of the active ingredient |
|---|---|---|---|
| vit A as retinol | (2667.7 IU) 800 µg | vitamin A palmitate 100000 IU/g | (2266.7 IU) 22.67 mg |
| | | beta carotene as betatab 10%E | (400 IU) 7.20 mg |
| vitamin D 3 | (200 IU) 5 µg | Cholecalciferol concentrate (water dispersible powder form) 100000 IU/g | 2.0 mg |
| Vitamin E | 10 mg | D-alpha-tocopherol acetate concentrate (powder form) 50 % | 29.80 mg |
| Vitamin K | 0.03 mg | vitamin K1 5% SD | 0.60 mg |
| Vitamin B1 | 4.20 mg | Thiamin monophosphoric acid ester chloride dihydrate | 6.59 mg |
| Vitamin B2 | 4.80 mg | Sodium riboflavin 5'-phosphate | 6.56 mg |
| Niacin | 54.0 mg | nicotinamide | 54.0 mg |
| Pantothenic acid | 18.0 mg | Calcium D-pantothenate | 19.57 mg |
| Vitamin B6 | 6.0 mg | Pyridoxine hydrochloride | 7.3 mg |
| Folic acid | 0.60 mg | Pteroylmonoglutamic acid | 0.60 mg |
| Vitamin B12 | 3.0 µg | Cyanocobalamin powder 0.1 % water soluble | 3.0 mg |
| Biotin | 30 µg | D-biotin | 30 µg |
| Vitamin C | 180 mg | Ascorbic acid fine powder | 180 mg |
| Calcium | 120 mg | Calcium carbonate | 295.61 mg |
| | | (see calcium pantothenate) | |
| Magnesium | 80 mg | Magnesium carbonate | 163 mg |
| | | Magnesium sulphate dihydrate | 159 mg |
| Iron | 14 mg | Ferric pyrophosphate | 56 mg |
| copper | 0.9 mg | Copper citrate 2.5 hydrate | 2.57 mg |
| iodine | 0.075 mg | Potassium iodine | 0.098 mg |
| zinc | 8 mg | Zinc citrate trihydrate | 25.63 mg |
| manganese | 1.8 mg | Manganese sulphate monohydrate | 5.54 mg |
| potassium | 20.4 mg | Potassium chloride | 38.96 mg |
| selenium | 50 µg | Sodium selenate anhydrous | 0.12 mg |
| chromium | 0.025 mg | Chromium chloride hexahydrate | 0.128 mg |
| molybdenum | 0.045 mg | Sodium molybdate dihydrate | 0.113 mg |
| Coenzyme Q10 | 3.0 mg | Coenzyme Q10 10 % | 30 mg |
and further excipients.

3. Oral nutritional supplement of claim 1, wherein the copper salt is a hemipentahydrate.

4. Oral nutritional supplement of claims 1 or 3, wherein the amount of the iron source is from 1 to 60 mg based on iron.

5. Oral nutritional supplement of any of claims 1, 3 or 4, wherein the amount of copper citrate is from 0.1 to 2 mg, preferably 0.3 to 1.1 mg based on copper.

6. Oral nutritional supplement of any of claims 1, 3, 4 or 5 comprising at least one further mineral and/or vitamin.

7. Oral nutritional supplement of any of claims 1, 3, 4, 5 or 6 comprising in addition iron fumarate 60% Descote.

8. Oral nutritional supplement of claim any of claims 1, 3, 4, 5, 6 or 7 which is a chewable tablet, an orodispersible tablet or an effervescent formulation.

9. Oral nutritional supplement of claim 8 which is an fast disintegrating orodispersible tablet having a disintegration time of less equal than 100 sec.

10. Process for manufacturing of the oral nutritional supplement according to claim 8 to 9.

11. Oral nutritional supplement according to claims 1 to 9 for the treatment of iron deficiency symptoms.

## Patentansprüche

1. Orale Nahrungsergänzung ohne Geschmacksmaskierungstechnologie, umfassend eine Eisenquelle und eine Kupferquelle, wobei die Eisenquelle Carbonyleisen ist und die Kupferquelle Kupfercitrat ist.

2. Orale Nahrungsergänzung ohne Geschmacksmaskierungstechnologie, umfassend eine Eisenquelle und eine Kupferquelle, wobei die Eisenquelle Eisenpyrophosphat ist und die Kupferquelle Kupfercitrat in einer Brausetablette von etwa 4800 mg mit der folgenden Wirkstoffzusammensetzung ist:
| Wirkstoffe | Menge basierend auf dem Wirkstoff | Form des Wirkstoffs | Menge der Form des Wirkstoffs |
|---|---|---|---|
| Vit. A als Retinol | (2667,7 IE ) 800 µg | Vitamin-A-Palmitat 100000 IE/g | (2266,7 IE) 22,67 mg |
| | | beta-Carotin als Betatab 10%E | (400 IE) 7,20 mg |
| Vitamin D3 | (200 IE) 5 µg | Cholecalciferolkonzentr at (in Wasser dispergierbare Pulverform) 100000 IE/g | 2,0 mg |
| Vitamin E | 10 mg | D-alpha-tocopherolacetatkonzent rat (Pulverform) 50 % | 29,80 mg |
| Vitamin K | 0, 03 mg | Vitamin K1 5 % Standardabweichung | 0,60 mg |
| Vitamin B1 | 4,20 mg | Thiaminmonophosphorsäur eesterchloriddihydrat | 6, 59 mg |
| Vitamin B2 | 4,80 mg | Natriumriboflavin-5'-phosphat | 6,56 mg |
| Niacin | 54,0 mg | Nicotinamid | 54,0 mg |
| Pantothensä ure | 18,0 mg | Calcium D-pantothenat | 19,57 mg |
| Vitamin B6 | 6 , 0 mg | Pyridoxinhydrochlorid | 7,3 mg |
| Folsäure | 0,60 mg | Pteroylmonoglutaminsäur e | 0, 60 mg |
| Vitamin B12 | 3,0 µg | Cyanocobalaminpulver 0,1 % wasserlöslich | 3,0 mg |
| Biotin | 30 µg | D-Biotin | 30 µg |
| Vitamin C | 180 mg | Ascorbinsäure feines Pulver | 180 mg |
| Calcium | 120 mg | Calciumcarbonat | 295,61 mg |
| | | (siehe Calciumpantothenat) | |
| Magnesium | 80 mg | Magnesiumcarbonat | 163 mg |
| | | Magnesiumsulfatdihydrat | 159 mg |
| Eisen | 14 mg | Eisenpyrophosphat | 56 mg |
| Kupfer | 0,9 mg | Kupfercitrat-2,5-Hydrat | 2,57 mg |
| Iod | 0,075 mg | Kaliumiod | 0,098 mg |
| Zink | 8 mg | Zinkcitrattrihydrat | 25,63 mg |
| Mangan | 1, 8 mg | Mangansulfatmonohydrat | 5,54 mg |
| Kalium | 20,4 mg | Kaliumchlorid | 38,96 mg |
| Selen | 50 µg | Natriumselenat wasserfrei | 0,12 mg |
| Chrom | 0,025 mg | Chromchloridhexahydrat | 0,128 mg |
| Molybdän | 0,045 mg | Natriummolybdatdihydrat | 0,113 mg |
| Coenzym Q10 | 3,0 mg | Coenzym Q10 10 % | 30 mg |
und weitere Trägerstoffe.

3. Orale Nahrungsergänzung nach Anspruch 1, wobei das Kupfersalz ein Hemipentahydrat ist.

4. Orale Nahrungsergänzung nach Anspruch 1 oder 3, wobei die Menge der Eisenquelle bezogen auf das Eisen von 1 bis 60 mg beträgt.

5. Orale Nahrungsergänzung nach einem der Ansprüche 1, 3 oder 4, wobei die Menge des Kupfercitrats bezogen auf das Kupfer von 0,1 bis 2 mg, vorzugsweise 0,3 bis 1,1 mg beträgt.

6. Orale Nahrungsergänzung nach einem der Ansprüche 1, 3, 4 oder 5, umfassend mindestens ein weiteres Mineral und/oder Vitamin.

7. Orale Nahrungsergänzung nach einem der Ansprüche 1, 3, 4, 5 oder 6, umfassend außerdem Eisenfumarat 60 % Descote.

8. Orale Nahrungsergänzung nach einem der Ansprüche 1, 3, 4, 5, 6 oder 7, die eine Kautablette, eine Schmelztablette oder eine Brausetablettenformulierung ist.

9. Orale Nahrungsergänzung nach Anspruch 8, die eine schnell auflösende Schmelztablette mit einer Auflösungszeit von kleiner gleich 100 Sekunden ist.

10. Verfahren zum Herstellen der oralen Nahrungsergänzung nach Anspruch 8 bis 9.

11. Orale Nahrungsergänzung nach Anspruch 1 bis 9 zur Behandlung von Eisenmangelsymptomen.

## Revendications

1. Supplément nutritionnel oral sans technologie de masquage de goût comprenant une source de fer et une source de cuivre, **caractérisé en ce que** la source de fer est du carbonyl-fer, et la source de cuivre est du citrate de cuivre.

2. Supplément nutritionnel oral sans technologie de masquage de goût comprenant une source de fer et une source de cuivre, **caractérisé en ce que** la source de fer est du pyrophosphate ferrique, et la source de cuivre est du citrate de cuivre dans un comprimé effervescent d'environ 4800 mg avec la composition suivante de composants actifs :
| Substances actives | Quantité sur la base de la substance active | Forme de la substance active | Quantité de la forme de la substance active |
|---|---|---|---|
| Vit. A sous forme de rétinol | (2667,7 UI) 800 µg | Palmitate de vitamine A 100000 UI/g | (2266,7 UI) 22,67 mg |
| | | bêta-carotène sous forme de betatab 10%E | (400 UI) 7,20 mg |
| Vitamine D3 | (200 UI) | Concentré de | 2,0 mg |
| | 5 µg | cholécalciférol (forme de poudre dispersible dans l'eau) 100000 UI/g | |
| Vitamine E | 10 mg | Concentré d'acétate de D-alpha-tocophérol (forme de poudre) 50 % | 29,80 mg |
| Vitamine K | 0 , 0 3 mg | vitamine K1 5 % SD | 0,60 mg |
| Vitamine B1 | 4,20 mg | Chlorure dihydraté d'ester d'acide monophosphorique de thiamine | 6,59 mg |
| Vitamine B2 | 4,80 mg | Riboflavine 5'-phosphate de sodium | 6,56 mg |
| Niacine | 54,0 mg | Nicotinamide | 54,0 mg |
| Acide pantothénique | 18,0 mg | D-Pantothénate de calcium | 19,57 mg |
| Vitamine B6 | 6 , 0 mg | Chlorhydrate de pyridoxine | 7,3 mg |
| Acide folique | 0,60 mg | Acide ptéroyl-monoglutamique | 0,60 mg |
| Vitamine B12 | 3,0 µg | Cyanocobalamine en poudre à 0,1 % hydrosoluble | 3,0 mg |
| Biotine | 30 µg | D-Biotine | 30 µg |
| Vitamine C | 180 mg | Acide ascorbique en poudre fine | 180 mg |
| Calcium | 120 mg | Carbonate de calcium | 295,61 mg |
| | | (voir pantothénate de calcium) | |
| Magnésium | 80 mg | Carbonate de magnésium | 163 mg |
| | | Sulfate de magnésium dihydraté | 159 mg |
| Fer | 14 mg | Pyrophosphate ferrique | 56 mg |
| Cuivre | 0,9 mg | Citrate de cuivre 2,5 hydrate | 2,57 mg |
| Iode | 0,075 mg | Iodure de potassium | 0,098 mg |
| Zinc | 8 mg | Citrate de zinc trihydraté | 25,63 mg |
| Manganèse | 1,8 mg | Sulfate de manganèse monohydraté | 5,54 mg |
| Potassium | 20,4 mg | Chlorure de potassium | 38,96 mg |
| Sélénium | 50 µg | Sélénate de sodium anhydre | 0,12 mg |
| Chrome | 0,025 mg | Chlorure de chrome hexahydraté | 0,128 mg |
| Molybdène | 0,045 mg | Molybdate de sodium dihydraté | 0,113 mg |
| Coenzyme Q10 | 3,0 mg | Coenzyme Q10 10 % | 30 mg |
et d'autres excipients.

3. Supplément nutritionnel oral de la revendication 1, dans lequel le sel de cuivre est un hémipentahydrate.

4. Supplément nutritionnel oral des revendications 1 ou 3, la quantité de la source de fer étant de 1 à 60 mg sur la base du fer.

5. Supplément nutritionnel oral de l'une quelconque des revendications 1, 3 ou 4, la quantité de citrate de cuivre étant de 0,1 à 2 mg, de préférence de 0,3 à 1,1 mg sur la base du cuivre.

6. Supplément nutritionnel oral de l'une quelconque des revendications 1, 3, 4 ou 5 comprenant au moins un autre minéral et/ou vitamine.

7. Supplément nutritionnel oral de l'une quelconque des revendications 1, 3, 4, 5 ou 6 comprenant en outre du fumarate de fer à 60 % Descote.

8. Supplément nutritionnel oral de l'une quelconque des revendications 1, 3, 4, 5, 6 ou 7 qui est un comprimé à mâcher, un comprimé orodispersible ou une formulation effervescente.

9. Supplément nutritionnel oral de la revendication 8 qui est un comprimé orodispersible à désintégration rapide ayant un temps de désintégration inférieur ou égal à 100 s.

10. Procédé pour fabriquer le supplément nutritionnel oral selon la revendication 8 à 9.

11. Supplément nutritionnel oral selon les revendications 1 à 9 pour le traitement de symptômes de déficience en fer.
